(19) Europäisches Patentamt
European Patent Office
Office européen des brevets

(11) Publication number: 0 235 864 B1

# (12) EUROPEAN PATENT SPECIFICATION

(45) Date of publication of the patent specification: 16.05.90

(51) Int. Cl.[5]: **C07C 51/14**, C07C 67/38, C07C 51/56, C07C 53/122, C07C 69/24, C07C 69/608

(21) Application number: 87200329.8

(22) Date of filing: 25.02.87

## (54) Process for the carbonylation of olefinically unsaturated compounds with a palladium catalyst.

(30) Priority: 28.02.86 GB 8605034

(43) Date of publication of application: 09.09.87 Bulletin 87/37

(45) Publication of the grant of the patent: 16.05.90 Bulletin 90/20

(84) Designated Contracting States: AT BE CH DE ES FR GB GR IT LI LU NL SE

(56) References cited:
EP-A- 0 055 875
EP-A- 0 106 379
EP-A- 0 186 228
EP-A- 0 190 473
EP-A- 0 198 521
GB-A- 1 066 772
US-A- 3 501 518

(73) Proprietor: SHELL INTERNATIONALE RESEARCH MAATSCHAPPIJ B.V., Carel van Bylandtlaan 30, NL-2596 HR Den Haag(NL)

(72) Inventor: Drent, Eit, Badhuisweg 3, NL-1031 CM Amsterdam(NL)

(74) Representative: Aalbers, Onno et al, P.O. Box 302, NL-2501 CH The Hague(NL)

EP 0 235 864 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a process for the carbonylation of an olefinically unsaturated compound with carbon monoxide in the presence of water, an alcohol and/or a carboxylic acid.

It is known from European Patent Application 106 379 that olefinically unsaturated compounds are carbonylated at a high reaction rate with carbon monoxide in the presence of water, an alcohol and/or a carboxylic acid, a palladium catalyst, at least 5 mol of a triarylphosphine per gram atom of palladium, and an acid having a $pK_a$ below 2, except hydrohalogenic and carboxylic acids. Table B in said patent application shows that modifying this known process by using an acid having a $pK_a$ higher than 2 (for example orthophosphoric acid or benzenephosphonic acid) or a carboxylic acid (for example trifluoroacetic acid or acetic acid) results in a low reaction rate.

It has now surprisingly been found that in the carbonylation of olefinically unsaturated compounds the reaction rate can be very much enhanced by the presence of an acid defined more closely hereinafter and of at least one mol of such an acid per mol of triarylphosphine.

Accordingly, the invention provides a process for the carbonylation of an olefinically unsaturated compound with carbon monoxide in the presence of water, an alcohol and/or a carboxylic acid, which process is carried out in the presence of a homogeneous catalytic system prepared by combining:-

a) a palladium(II) compound;
b) at least 5 mol of a phosphine of the general formula I

$$R^1 - \overset{R^2}{\underset{|}{P}} - R^3 \qquad (I)$$

in which $R^1$, $R^2$ and $R^3$ each individually represent an optionally substituted aryl group, per gram atom of palladium, and
c) at least 1 mol of a non-carboxylic acid having a $pK_a$ greater than 2 and/or of a sterically hindered carboxylic acid having a $pK_a$ below 4.5, per mol of phosphine of the general formula I, the $pK_a$ values measured at 18 °C in aqueous solution.

Examples of non-carboxylic acids having a $pK_a$ greater than 2 and which are preferably used in the process according to the present invention are benzenephosphonic acid ($pK_a$=2.5), 2-bromobenzenephosphonic acid ($pK_a$=2.1) and orthophosphoric acid ($pK_a$=2.1). Another example of such acids is arsenic acid.

The carboxylic acid must be sterically hindered which means that atoms or groups of atoms are present which interfere with one another, thus counteracting esterification of the acid. Examples of such acids are 2,4,6-trimethylbenzoic acid and 2,6-dimethylbenzoic acid. It is preferred to apply sterically hindered carboxylic acids having a $pK_a$ - measured at 18 °C in aqueous solution - below 2. Among the sterically hindered carboxylic acids preference is given to the sterically hindered benzoic acids. Very good results have been obtained with 2,6-dichlorobenzoic acid ($pK_a$=1.5). Other examples of sterically hindered benzoic acids are 2,6-difluorobenzoic acid, 2,4,6-trifluorobenzoic acid, 2,4,6-trichlorobenzoic acid, 2,6-dibromobenzoic acid, 2,4,6-tribromobenzoic acid, 2,6-diiodobenzoic acid and 2,4,6-triiodobenzoic acid. Modification of the process according to the present invention by replacing a sterically hindered carboxylic acid having a $pK_a$ below 4.5 with a sterically hindered carboxylic acid having a $pK_a$ above 4.5 results in an extremely low rate of carboxylation.

The upper limit for the molar ratio non-carboxylic acid having a $pK_a$ greater than 2 and/or sterically hindered carboxylic acid to phosphine of the general formula I is not critical. This molar ratio is preferably not more than 50, but the use of molar ratios higher than 50, for example up to 150, is not excluded.

The olefinically unsaturated compound may be an unsubstituted or a substituted alkene or cycloalkene preferably having in the range of from 2 to 30, and in particular 2 to 20, carbon atoms and preferably in the range of from 1 to 3 double bonds. The alkene or cycloalkene may be substituted, for instance, with one or more halogen atoms or cyano, ester, alkoxy, hydroxyl, carboxyl or aryl groups. If the substituents are not inert under the reaction conditions, the carbonylation reaction may be accompanied with other reactions. For instance, the carbonylation of allyl alcohol is accompanied with esterification of the hydroxy group. Examples of suitable olefinic compounds are ethene, propene, 1-butene, 2-butene, isobutene, the isomeric pentenes, hexenes, octenes and dodecenes, 1,5-cyclooctadiene, cyclododecene, 1,5,9-cyclododecatriene, allyl alcohol, methyl acrylate, ethyl acrylate, methyl methacrylate, acrylonitrile, acrylamide, N,N-dimethylacrylamide, vinyl chloride, allyl chloride, acrolein, oleic acid, methyl allyl ether and styrene.

The alcohols or carboxylic acids used in the process according to the invention may be aliphatic, cycloaliphatic or aromatic and may be substituted with one or more substituents, such as mentioned hereinbefore in connection with the olefinically unsaturated compounds to be used as starting material. The alcohol may therefore also be a phenol. The alcohols or carboxylic acids preferably contain not more than 20 carbon atoms. Examples of suitable alcohols or carboxylic acids are methanol, ethanol, propanol, isobutanol, tert-butyl alcohol, stearyl alcohol, benzyl alcohol, cyclohexanol, allyl alcohol, chlorocapryl alcohol, ethylene glycol, 1,2-propanediol, 1,4-butanediol, glycerol, polyethylene glycol, 1,6-hexanediol, phenol, cresol, acetic acid, propionic acid, butyric acid, caproic acid, trimethylacetic acid, benzoic acid, caprylic acid, succinic acid, adipic acid and hydroxycaproic acid. Special preference is given to alkanols and carboxylicacids having in the range of from 1 to 10 carbon atoms per molecule. If the alcohol or the carboxylic acid has more than one hydroxyl group or carboxyl group, different products may be formed, depending on the molar ratios existing between the reagents. For instance, depending on the quantity of olefinically unsaturated compound used, either a mono-ester or a diester may be produced from glycerol.

The process according to the present invention is particularly suitable for the preparation of methyl propionate, starting from ethylene, carbon monoxide and methanol.

The products formed in the process according to the invention may be further reacted if desired. For instance, the carbonylation of an olefin, when conducted in the presence of water, yields a carboxylic acid which, by reaction with a further quantity of olefin, may form an anhydride of a carboxylic acid. When the carbonylation is carried out in the presence of an alcohol, it yields an ester which, when water is present as well, may hydrolyze to form an acid and an alcohol, each of which may again react with an olefin. When the carbonylation is carried out in the presence of a carboxylic acid, it yields an anhydride of a carboxylic acid which, when water is present as well, may hydrolyze to form one or more carboxylic acids which in their turn may react with a further quantity of olefin.

Reaction of an alkanecarboxylic acid having n + 1 carbon atoms per molecule with an olefin having n carbon atoms per molecule yields the symmetrical anhydride of the alkanecarboxylic acid having n + 1 carbon atoms per molecule. This anhydride may optionally be hydrolyzed, half of the carboxylic acid formed may be collected as a product and the other half recycled to the carbonylation reactor. The process thus leads to the conversion of an olefin having n carbon atoms into a carboxylic acid having n + 1 carbon atoms.

Suitable homogeneous catalysts are the salts of palladium(II) with, for instance, nitric acid, sulphuric acid or alkanecarboxylic acids having not more than 12 carbon atoms per molecule. Salts of hydrohalogenic acids may, in principle, be used as well, but they have the drawback that the halogen ion may have a corrosive effect. A catalyst used by preference is palladium(II) acetate. Moreover, palladium complexes may be used, for instance palladium acetylacetonate, tetrakistriphenylphosphinepalladium, bis-trio-tolylphosphinepalladium acetate or bis-triphenylphosphinepalladium sulphate.

The quantity of palladium catalyst is not critical. Preference is given to the use of quantities in the range between $10^{-5}$ and $10^{-1}$ gram atom palladium per mol of olefinically unsaturated compound.

The substituted or unsubstituted aryl groups $R^1$, $R^2$ and $R^3$ of the phosphine $PR^1R^2R^3$ preferably contain not more than 18, in particular in the range of from 6 to 14 carbon atoms. Examples of suitable $R^1$, $R^2$ and $R^3$ groups are the naphthyl group and in particular the phenyl group. Suitable substituents are halogen atoms and alkyl, aryl, alkoxy, carboxy, carbalkoxy, acyl, trihalogenmethyl, cyano, dialkylamino, sulphonylalkyl and alkanoyloxy groups.

Examples of suitable phosphines are tri-p-tolylphosphine, tri-p-methoxyphenylphosphine, o-diphenylphosphinobenzoic acid and in particular triphenylphosphine. The phosphine is used in a quantity of at least 5 mol, preferably in the range of from 10 to 150 mol per gram atom of palladium. If the palladium catalyst already contains phosphine, this should be taken into account when calculating the amount of phosphine to be used.

In the process according to the invention the carbon monoxide may be used pure or diluted with an inert gas, such as nitrogen, noble gases or carbon dioxide. Generally the presence of more than 10 %v of hydrogen is undesirable, since under the reaction conditions it may cause hydrogenation of the olefinic compound. Generally preference is given to the use of carbon monoxide or a carbon monoxide-containing gas which contains less than 5 %v of hydrogen.

The carbonylation according to the invention is preferably carried out at a temperature in the range of from 50 to 200 °C, in particular 75 to 150 °C. The overall pressure preferably lies in the range of from 1 to 100, in particular 20 to 75, bar.

The molar ratio of the olefinically unsaturated compound to water, alcohol or carboxylic acid is not critical. The molar ratio between hydroxy groups and olefinic double bonds may lie for instance in the range of from 0.1 : 1 to 10 : 1. When using a mono-olefin and either water, a monohydric alcohol or a monobasic acid, preference is usually given to the use of an excess of the hydroxy compound mentioned. However, when using a polyhydric alcohol or a polybasic acid to prepare a polyester or a polyanhydride, it will generally be necessary to use an excess of olefinic compound.

The process according to the invention may be carried out batchwise, continuously or semi-continuously. Generally there is no need for the use of a solvent since usually there will be an excess of one of the reactants - for instance the alcohol - which may serve as a solvent as well. If required, however, a solvent may be used, for instance a sulphoxide such as dimethyl sulphoxide or diethyl sulphoxide; a sulphone such as diisopropyl sulphone or tetrahydrothiophene 1,1-dioxide (also referred to as "sulfolane"); ethers such as diethyl ether, tetrahydrofuran, 3,6-dioxaoctane (diethyl ether of diethylene glycol), methyl tert-butyl ether, 1,4-dioxane, anisole, 2,5,8-trioxanonane (dimethyl ether of diethylene glycol, also referred to as "diglyme"), diphenyl ether and diisopropyl ether; hydrocarbons such as hexane, cyclohexane, octane, benzene, toluene, o-xylene, m-xylene, p-xylene, ethylbenzene and cumene; halogenated hydrocarbons such as chloroform, 1,2-dichloroethane, perfluoroalkanes, chlorobenzene, 1,2-dichlorobenzene, 1,3-dichlorobenzene and 1,4-dichlorobenzene; N,N-dialkyl substituted amides such as N,N-dimethylformamide and N-methylpyrrolidone; esters such as methyl propionate, ethyl acetate and methyl benzoate; nitro compounds such as nitrobenzene. The primary reaction product of the carbonylation reaction may also be used as a solvent.

The following Examples further illustrate the invention.

Example 1

A 300-ml magnetically stirred Hastelloy C autoclave ("Hastelloy" is a trade mark) was charged with methanol (50 ml), palladium(II) acetate (0.1 mmol), triphenylphosphine (3 mmol) and orthophosphoric acid (10 mmol). The autoclave was flushed with carbon monoxide, filled with ethylene until a pressure of 20 bar was obtained and then with carbon monoxide until a partial pressure thereof of 30 bar was obtained, sealed and heated to a temperature of 120 °C. The contents of the autoclave were analysed by gas-liquid chromatography; the reaction rate was 700 g methyl propionate per g palladium per hour.

Example 2

An experiment was carried out as described in Example 1, charging the autoclave with the following materials:-

| | | | |
|---|---|---|---|
| methanol | 50 ml | 2,6-dichlorobenzoic acid | 10 mmol |
| palladium(II) acetate | 0.1 mmol | ethylene | 20 bar |
| tri(p-methoxyphenyl) phosphine | 2 mmol | carbon monoxide | 30 bar |

The reaction rate was 1000 g methyl propionate per g palladium per hour.

Example 3

An experiment was carried out as described in Example 1, charging the autoclave with the following materials:-

| | | | |
|---|---|---|---|
| methanol | 50 ml | benzenephosphonic acid | 10 mmol |
| palladium(II) acetate | 0.1 mmol | ethylene | 20 bar |
| triphenylphosphine | 2 mmol | carbon monoxide | 30 bar |

The reaction rate was 800 g methyl propionate per g palladium per hour.

Example 4

An experiment was carried out as described in Example 2 with the exception that 2,6-dichlorobenzoic acid (10 mmol) was replaced with 2,4,6-trimethylbenzoic acid (10 mmol, $pK_a$ below 4.5).

The reaction rate was 400 g methyl propionate per g palladium per hour.

Comparative Experiment

An experiment was carried out as described in Example 2 with the exception that 2,6-dichlorobenzoic acid (10 mmol) was replaced with pivalic acid (10 mmol, $pK_a$=5.0).

The reaction rate was less than 5 g methyl propionate per g palladium per hour.

## Claims

1. A process for the carbonylation of an olefinically unsaturated compound with carbon monoxide in the presence of water, an alcohol and/or a carboxylic acid, which process is carried out in the presence of a homogeneous catalytic system prepared by combining:-

a) a palladium(II) compound;
b) at least 5 mol of a phosphine of the general formula I

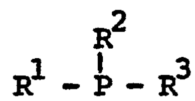 (I)

in which $R^1$, $R^2$ and $R^3$ each individually represent an optionally substituted aryl group, per gram atom of palladium, and
c) at least 1 mol of a non-carboxylic acid having a $pK_a$ greater than 2 and/or of a sterically hindered carboxylic acid having a $pK_a$ below 4.5, per mol of phosphine of the general formula I, the $pK_a$ values measured at 18 °C in aqueous solution.

2. A process as claimed in claim 1 in which the non-carboxylic acid is benzenephosphonic acid.

3. A process as claimed in claim 1 in which the non-carboxylic acid is orthophosphoric acid.

4. A process as claimed in any one of the preceding claims in which the sterically hindered carboxylic acid has a $pK_a$ – measured at 18 °C in aqueous solution – below 2.

5. A process as claimed in any one of the preceding claims in which the sterically hindered carboxylic acid is a benzoic acid.

6. A process as claimed in claim 5 in which the benzoic acid is 2,6-dichlorobenzoic acid.

7. A process as claimed in any one of the preceding claims in which not more than 50 mol of said non-carboxylic acid and/or of said sterically hindered carboxylic acid are used per mol of said phosphine.

8. A process as claimed in any one of the preceding claims in which from 10 to 150 mol of said phosphine per gram atom of palladium are used.

9. A process as claimed in any one of the preceding claims in which the aryl groups represented by $R^1$, $R^2$ and $R^3$ in the general formula I have in the range of from 6 to 14 carbon atoms.

10. A process as claimed in any one of the preceding claims in which the aryl groups are phenyl groups.

11. A process as claimed in any one of the preceding claims in which the palladium(II) compound is a palladium(II) salt of an alkanoic acid having not more than 12 carbon atoms per molecule.

12. A process as claimed in claim 11 in which the palladium(II) compound in palladium(II) acetate.

13. A process as claimed in any one of the preceding claims which is carried out at a temperature in the range of from 50 °C to 200 °C.

14. A process as claimed in any one of the preceding claims which is carried out at a total pressure in the range of from 1 to 100 bar.

**Patentansprüche**

1. Verfahren zur Carbonylierung einer olefinisch ungesättigten Verbindung mit Kohlenmonoxid in Anwesenheit von Wasser, eines Alkohols und/oder einer Carbonsäure, welches Verfahren in Gegenwart eines homogenen katalytischen Systems ausgeführt wird, das durch Vereinigen von:
a) einer Palladium(II)-Verbindung;
b) wenigstens 5 Mol eines Phosphins der allgemeinen Formel I

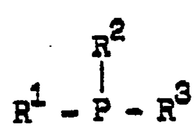 (I) ,

worin $R^1$, $R^2$ und $R^3$ jeweils unabhängig voneinander eine gegebenenfalls substituierte Arylgruppe bedeuten, je Grammatom Palladium, und
c) wenigstens 1 Mol einer Nicht-Carbonsäure mit einem $pK_a$-Wert von größer als 2 und/oder einer sterisch gehinderten Carbonsäure mit einem $pK_a$-Wert von unter 4,5, je Mol Phosphin der allgemeinen Formel I, wobei die $pK_a$-Werte bei 18°C in wäßriger Lösung gemessen werden, hergestellt worden ist.

2. Verfahren nach Anspruch 1, worin die Nicht-Carbonsäure Benzolphosphonsäure ist.

3. Verfahren nach Anspruch 1, worin die Nicht-Carbonsäure Orthophosphorsäure ist.

4. Verfahren nach einem der vorstehenden Ansprüche, worin die sterisch gehinderte Carbonsäure einen $pK_a$-Wert, gemessen bei 18°C in wäßriger Lösung, von unter 2 aufweist.

5. Verfahren nach einem der vorstehenden Ansprüche, worin die sterisch gehinderte Carbonsäure eine Benzoesäure ist.

6. Verfahren nach Anspruch 5, worin die Benzoesäure 2,6-Dichlorbenzoesäure ist:

7. Verfahren nach einem der vorstehenden Ansprüche, worin nicht mehr als 50 Mol der Nicht-Carbonsäure und/oder der sterisch gehinderten Carbonsäure je Mol Phosphin verwendet werden.

8. Verfahren nach einem der vorstehenden Ansprüche, worin von 10 bis 150 Mol Phosphin je Grammatom Palladium verwendet werden.

9. Verfahren nach einem der vorstehenden Ansprüche, worin die durch $R^1$, $R^2$ und $R^3$ in der allgemeinen Formel I dargestellten Arylgruppen 6 bis 14 Kohlenstoffatome aufweisen.

10. Verfahren nach einem der vorstehenden Ansprüche, worin die Arylgruppen Phenylgruppen sind.

11. Verfahren nach einem der vorstehenden Ansprüche, worin die Palladium(II)-Verbindung ein Palladium(II)-Salz einer Alkansäure mit nicht mehr als 12 Kohlenstoffatomen je Molekül ist.

12. Verfahren nach Anspruch 11, worin die Palladium(II)-Verbindung Palladium(II)-acetat ist.

13. Verfahren nach einem der vorstehenden Ansprüche, welches bei einer Temperatur im Bereich von 50°C bis 200°C ausgeführt wird.

14. Verfahren nach einem der vorstehenden Ansprüche, welches bei einem Gesamtdruck im Bereich von 1 bis 100 bar ausgeführt wird.

**Revendications**

1. Un procédé pour la carbonylation d'un composé oléfiniquement insaturé avec de l'oxyde de carbone en présence d'eau, d'un alcool et/ou d'un acide carboxylique, lequel procédé est mis en œuvre en présence d'un système catalytique homogène préparé en combinant:

a) un composé de palladium (II);

b) au moins 5 moles d'une phosphine de la formule générale I

$$R^1 - \underset{}{\overset{R^2}{\overset{|}{P}}} - R^3 \qquad (I)$$

où $R^1$, $R^2$ et $R^3$ représentent chacun individuellement un groupe aryle éventuellement substitué, par atome-gramme de palladium, et

c) au moins 1 mole d'un acide non-carboxylique ayant un $pK_a$ au-dessus de 2 et/ou d'un acide carboxylique comportant un empêchement stérique ayant un $pK_a$ au-dessous de 4,5, par mole de phosphine de la formule générale I, les valeurs de $pK_a$ étant mesurées à 18°C en solution aqueuse.

2. Un procédé selon la revendication 1, dans lequel l'acide non-carboxylique est de l'acide benzènephosphonique.

3. Un procédé selon la revendication 1, dans lequel l'acide non-carboxylique est de l'acide orthophosphorique.

4. Un procédé selon l'une quelconque des revendications précédentes, dans lequel l'acide carboxylique comportant un empêchement stérique a un pKa – mesuré à 18C en solution aqueuse – au-dessous de 2.

5. Un procédé selon l'une quelconque des revendications précédentes, dans lequel l'acide carboxylique comportant un empêchement stérique est un acide benzoïque.

6. Un procédé selon la revendication 5, dans lequel l'acide benzoïque est l'acide 2,6-dichlorobenzoïque.

7. Un procédé selon l'une quelconque des revendications précédentes, dans lequel on n'utilise pas plus de 50 moles de l'acide non-carboxylique et/ou de l'acide carboxylique comportant un empêchement stérique par mole de la phosphine.

8. Un procédé selon l'une quelconque des revendications précédentes, dans lequel on utilise de 10 à 150 moles de la phosphine par atome-gramme de palladium.

9. Un procédé selon l'une quelconque des revendications précédentes, dans lequel les groupes aryle représentés par $R^1$, $R^2$ et $R^3$ dans la formule générale I ont de 6 à 14 atomes de carbone.

10. Un procédé selon l'une quelconque des revendications précédentes, dans lequel les groupes aryle sont des groupes phényle.

11. Un procédé selon l'une quelconque des revendications précédentes, dans lequel le composé de palladium (II) est un sel de palladium (II) d'un acide alcanoïque n'ayant pas plus de 12 atomes de carbone par molécule.

12. Un procédé selon la revendication 11, dans lequel le composé de palladium (II) est de l'acétate de palladium (II).

13. Un procédé selon l'une quelconque des revendications précédentes, qui est mis en oœuvre à une température comprise entre 50°C et 200°C.

14. Un procédé selon l'une quelconque des revendications précédentes, qui est mis en oœuvre à une pression totale comprise entre 1 et 100 bars.